# EUROPEAN PATENT SPECIFICATION

(11) **EP 0 996 486 B1**
(45) Date of publication and mention of the grant of the patent: **21.01.2004**
(21) Application number: 98906735.0
(22) Date of filing: 12.02.1998
(51) Int. Cl.: A61N 5/06

(54) **DEVICE FOR EHF PUNCTURE (OR MMW THERAPY)**
VORRICHTUNG ZUR EHF PUNKTION (ODER THERAPIE MIT MILLIMETRISCHEN WELLENLÄNGEN)
DISPOSITIF DE THERAPIE PAR ONDES MILLIMETRIQUES

(30) Priority: 17.04.1997 AM 97029
(43) Date of publication of application: 03.05.2000
(73) Proprietor: Avagyan, Romen, Yerevan, 375009 (AM); Teppone, Michail, Tomilino Moscow obl., 140070 (RU)
(72) Inventor: AVAGYAN, Romen, Yerevan, 375009 (AM); TEPPONE, Michail, Moscow obl., 140070 (RU); TAUBE, Alex, Kiev, 252148 (UA)
(74) Representative: Hengelhaupt, Jürgen, Dipl.-Ing.
(86) International application number: PCT/AM1998/000002
(87) International publication number: WO 1998/047567

(56) References cited:
- EP-A- 0 145 176
- EP-A- 0 484 064
- US-A- 3 878 480

## Description

### 1. TECHNICAL FIELD

This invention relates to medicine, and particularly to reflexotherapy, and is designed for treatment of various diseases and dysfunctions in the organism.

### 2. BACKGROUND ART

Known is the method of microwave resonance therapy which consists in action on the biologically active zone (BAZ), selected by nosologic diagnosis, by means of extremely-high-frequency (EHF) electromagnetic radiation having continuous noiselike spectrum (see US Patent #5.152,286).

Known is also the method of microwave resonance therapy consisting in action on BAZ by a low-intensity EHF electromagnetic radiation at fixed frequencies (see N.D. Devyatkov et al., *Millimeter Waves and Their Role in Vital Activity,* Moscow, 1991, 169 pp.; *Methodical Recommendations for Application of MMW Therapy Under Various Nosologic Forms.* Approved by a Joint USSR Commission including, the State Committee on Science and Technology, USSR Academy of Sciences, and USSR Public Health Ministry representatives, November 29, 1991. Moscow, 1992, 90 pp. ).

Known is the method of reflexotherapy consisting in action by various physical factors on biologically active points (BAPs) and BAZes selected according to a bio-holographic law, or ECIWO (an Embryo Containing Information of the Whole Organism) biology (see Yingqing Zhang, *ECIWO Biology and Medicine: A New Theory of Conquering Cancer and a Completely New Acupuncture Theory,* Beijing, Neimenggu People's Press, 1987, 264 pp.). ECIWO biology includes such known microsystems as helix, scalp, nasal region, sole and palm surfaces, etc.

Known is also the method of electric acupuncture and diagnostics by a method due to R. Voll (see R. Voll, *Topografische Lage der Messpuncte der Electroacupunktur* .Bd. 1-4, Uelzen, 1976-80, pp. 156,168, 158, 147), in which the pulsed electric current is applied to "anomalous" BAPs (i.e. those BAPs whose electric conductivity is beyond the "normal corridor").

Known are the devices for reflexotherapy using the low-intensity ( non-thermal) level MMW electromagnetic radiation in the noiselike mode (see USSR IC # 1,703,103, dated Sep. 19, 1989; and RF Patent # 2,066.556, dated Jan. 24, 1994).

Known are also the devices for reflexotherapy using the low-intensity coherent radiation in MMW band, at fixed frequencies (see A. N. Balaba et al., *"Elektronika KVCh-03" Device,* Elektronnnaya Promyshlennost, 1988, no. 2, pp. 50-52; and Yu. V. Dedik, *"Yav-I" Device for EHF Therapy,* Elektonnaya Promyshlennost, 1988, no. 2, p. 53).

Furthermore, US-A-3 878 480 discloses a millimeter wave oscillator having the structural features of the first part of claim 1.

Known methods and devices thereto possess the following disadvantages:
- impossibility of combined action upon the BAPs and BAZes by coherent and noiselike EHF radiation;
- BAPs or BAZes selection is based on nosologic diagnosis;
- limited range of pulse modulation rates;
- impossible simultaneous tonification or sedation of several selected BAPs (or BAZes).

### 3. DISCLOSURE OF INVENTION

It is the aim of this invention to enhance the therapeutic effect and reduce the treatment time. The said aim is reached, according to this invention, by using a combined noiselike and coherent EHF radiation on BAPs (or BAZes) selected by a syndrome diagnosis of Traditional Chinese Medicine (TCM), or instrumental methods of functional diagnostics of the channels (meridians) and acupoints state.

The combined EHF radiation represents a sum of broadband noiselike and coherent radiations in the millimeter wave region, the frequencies of coherent component corresponding to therapeutic frequencies, particularly to molecular oxygen and/or water vapor absorption frequencies.

The suggested method of EHF puncture and the device thereto, according to the preferred embodiment of invention, provide the action on BAPs and BAZes either by a noiselike or combined EHF radiation.

Selection of BAPs (or BAZes) and exposure mode (time, frequency range, radiation power and type, modulation pulse rate) is based on TCM syndrome diagnosis or instrumental methods of functional diagnostics of the channels and acupoints state. The following instrumental methods of anomalous points detection are predominantly used: skin resistance variation methods (Voll, Nakatani, Bratu, Mora et al.); thermal sensitivity method (Akabane); acupoint imaging method using ACUVISION device. Measured data may be either processed by computer method (using e.g. MIDIN, MIF, PROGNOS, AMSAT, DOCTOR-A and other hardware/software systems). or not.

Depending on the revealed variations (based on description of normal and pathological state by TCM), the syndrome Chinese diagnosis is made, namely the symptoms of Excess or Deficiency, Heat or Cold, Interior or Exterior, Yin or Yang, disease localization in a particular Channel or Organ (e.g. Excessive Heat, Interior, in Stomach and Large Intestine). Disease localization (Channel or Organ), acuteness and the state of the affected Channel or Organ determine the BAPs (or BAZes) selected for EHF exposure and its mode.

### Exposure Modes

Exposure modes are selected as follows: sedate in Excess, tonify in Deficiency, cool in Heat and warm in Cold syndromes. In presence of Exterior syndrome EHF may be combined with acupuncture (at Heat syndrome) or moxibustion (at Cold syndrome). In presence of Interior syndrome EHF exposure may be combined with TCM syndrome medicines (i.e. tonifying or sedative remedies, warming or cooling, and possessing a specific taste and «warm» or «cold» category.

The effect modality is determined by the BAPs (BAZes) exposure time. Individual exposure time is determined either on basis of sensor reactions of patients or instrumental methods of functional diagnostics of the channels and acupoints state.
To obtain the stimulating effect, the exposure time must be not more than 2-3 minutes. In this case it is necessary to wait for the first specific sensor reactions (usually comforting) and their growth to maximum. When BAPs are to be tonified, sensor response is sufficiently fast.

To obtain the sedative effect, the exposure time may be 30 minutes, or more. In this case it is necessary to wait for specific sensor reactions (usually discomforting), their intensity growth, stabilization, and disappearance.

To obtain the warming effect, in the case of Cold syndrome, the exposure time must be not more than 0.5-1.0 minutes. Some patients reveal the warmth sensation in the exposure area, or in other parts of organism. In this case pulse rate may rise from 2-3 beats per breath (Cold syndrome) up to 5 beats per breath, which is normal pulse to breath rate. In the case of Cold syndrome, EHF puncture may be combined with moxibustion (heating) of BAPs using special sticks filled with *Artemisia Vulgaris.*

To obtain the cooling effect, in the case of Heat syndrome, the exposure time may be 30 minutes, or more. Patients reveal the cooling sensation of face, abdomen, etc. Pulse is rarefied from 6-8 beats per breath (Heat syndrome) down to 5 beats per breath. To enhance the cooling effect, EHF puncture may be combined with acupuncture.

Considering the individual sensitivity to MMW exposure it is necessary to observe that though the specific sensations allows to individualize the BAPs (BAZes) exposure time , one should also note that the exposure time for the patients having white skin must be less than for those with a dark and well-tanned skin. In other words, the patients revealing the Yin Deficiency in Lung require less exposure time than those having Yang Deficiency in Lung.

TCM recommendations should be taken into account when the exposure part of organism is selected. Thus the right part of organism should be sedated in spring and summer seasons, and left part of organism should be sedated in autumn and winter seasons.

Though simultaneous exposure of several BAPs (BAZes) is possible, it is preferable to apply sequential exposures at first of BAPs requiring tonification and then of BAPs requiring sedation, except in some special cases. This is explained by specific quality of MMW exposure to initiate sensor reactions in patients allowing to individualize the exposure duration.

### EHF Puncture Application to Microsystems

According to ECIWO biology, the structures related with separate parts of the whole organism may be found on any small area of the organism. Thus exposure of various BAPs within these microsystems may result in therapeutic effect in corresponding organs (see Yingqing Zhang, *ECIWO Biology and Medicine: A New Theory of Conquering Cancer and a Completely New Acupuncture Theory,* Beijing, Neimenggu People's Press, 1987, 264 pp.).

BAPs (BAZes) having a varied electric conductivity or sensitivity (painful points) are determined in any microsystem, such as helix, scalp, nasal region, sole and palm surfaces, etc. EHF radiation antenna is applied to these «anomalous» BAPs (BAZes). Tonification effect is reached during the first 2-3 minutes and is accompanied by a fast creation and growth of comfort sensations intensity in domain of conditional localization of pathogenic organ. Sedative effect is reached after 15 minutes of exposure or more, and is accompanied by a slow creation, intensity growth and stabilization, and therefore disappearance of discomfort sensations.

As an example, EHF puncture is applicable to BAPs on sole and palm surfaces selected by methods due to *Park Jae Woo (Su Jok).* According to one of these methods, the thumb or toe corresponds to head and neck, the 2^{nd} and 5^{th} fingers correspond to arms, while 3^{rd} and 4^{th} fingers correspond to legs. Other parts of sole and palm correspond to spine, thorax and abdomen. According to the other *Su Jok* method, the classical channels are projected on palms and soles (the so-called *Belle-Meridians*).

EHF puncture applied to BAPs selected by the first *Su Jok* method is identical to the method described earlier in this invention which selects BAPs having a varied electric conductivity or sensitivity (painful points). In the second *Su Jok* method, both classical diagnostics and one described by *Park Jae Woo* is applicable. The desired *Belle -Meridians* points are selected on basis of revealed syndrome diagnosis. Tonification effect is reached during the first 2-3 minutes and is accompanied by a fast creation and growth of comfort sensations intensity in domain of conditional localization of pathogenic organ. Sedative effect is reached after 15 minutes of exposure or more , and is accompanied by a slow creation, intensity growth and stabilization, and therefore disappearance of discomfort sensations.

### EHF Puncture Application to Pulse Point Areas

Pulse area represents the same diagnostic and therapeutic microsystem as helix, scalp, nasal region, sole and palm surfaces, etc. Localization and character of pathologies are determined either by classical TCM diagnostic methods, or by a simpler pulse diagnostic method based on the patient's sensor reactions originating in palpation of pulse positions. Pulse position related with the initial pathology is selected, with center over the ray artery projection.

EHF puncture is applied to one of six classical pulse position points *(Tsung, Guan* and *Chi)* corresponding to the revealed pathology. The dielectric antenna of EHF generator is positioned over the selected pulse point and sensor reactions of patient are observed. Fast creation of comfort sensations (outside the pulse area) reflects the tonifying effect of channels existing in Deficiency state, while slow creation and disappearance of discomfort sensations reflects the sedative effect of channels existing in Excess state.

### EHF Puncture Application to Voll Points

Using the devices based on *Voll* diagnostic method, «anomalous» points are selected, with account of «pointer drop» phenomenon. EHF puncture is applied to those points having the largest deflection from normal state, with tonification of points having the lowest indications and sedation of points having the highest indications of *Voll* device. Tonification effect is reached during the first 2-3 minutes, while sedative effect is reached after 15 minutes of exposure or more. Individual selection of exposure time is based on sensor reactions created during the treatment procedure. Repeated *Voll* diagnostics is made after treatment for therapy control.

### 4. BRIEF DESCRIPTION OF DRAWINGS

Fig. 1 illustrates the structural diagram of the device for EHF puncture.
Fig 2 illustrates the structure of EHF oscillator.
Fig. 3 illustrates the structural diagram of extremely broadband device for EHF puncture including two EHF **(G1, G2)** oscillators operating in different parts of millimeter waveband (MMW), with p-i-n attenuators **(A1, A2)** of said oscillators having their outputs connected via summator (E) to directed emitter.
Fig. 4 illustrates the directed emitter in form of a horn antenna used for BAZes exposure.
Fig. 5 illustrates the same directed emitter which additionally includes a dielectric rod antenna for BAPs exposure.
Fig. 6 illustrates the same directed emitter which additionally includes a flexible dielectric waveguide with power divider **(D)** for simultaneous exposure of two BAPs.
Fig. 7 illustrates the frontal view of the same directed emitter which additionally includes coherent and incoherent sources of infrared and visible emission.
Fig. 8 illustrates the cross-sectional view of emitter shown in Fig. 7.

### 5. BEST MODE FOR CARRYING OUT THE INVENTION

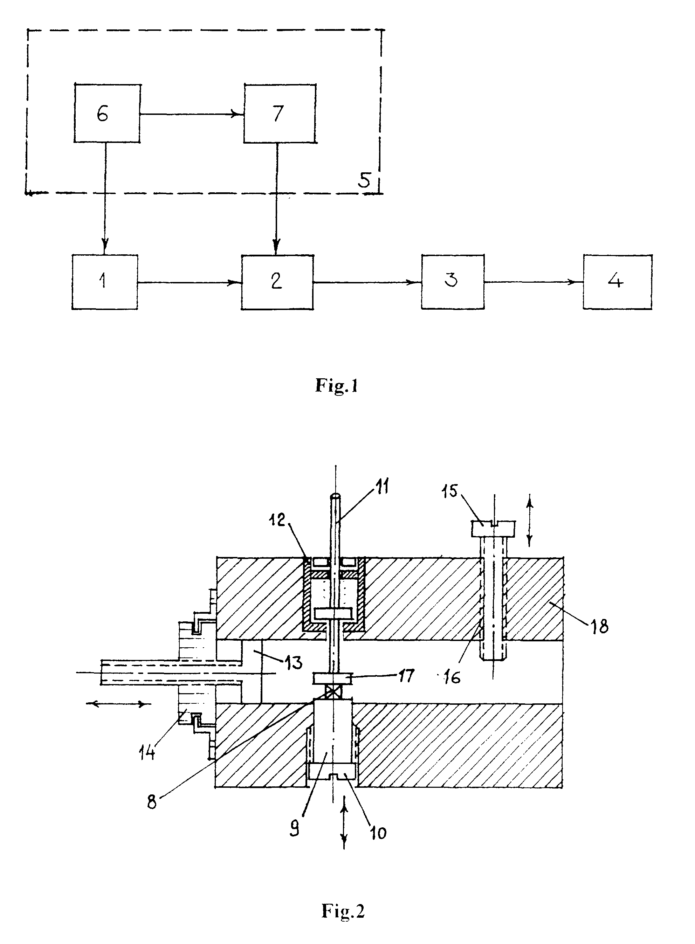

Fig. 1 illustrates the structural diagram of the device according to the preferred embodiment of invention. The device includes an EHF oscillator **1** whose output is fed through a p-i-n attenuator **2** and impedance transformer **3** to an emitter **4**. Microprocessor control unit **5** provides the required power-supply modes for EHF oscillator **1** and p-i-n attenuator **2** from the stabilized current sources **5** and **6**.

Fig 2 illustrates the structure of EHF oscillator **1**, including a section of waveguide **18** with an avalanche diode **8** (active element) mounted therein on a heat sink **9** and provided with adjustment screw **10**, to displace the diode **8** in the cross-section plane of the waveguide **18**. Power to the avalanche diode **8** is supplied through a post **11**, a disk resonator **17** and a filter **12** disposed in the wall and cavity of the waveguide **18**. One side of the waveguide **18** has a short-circuiting plunger **13** mounted therein and provided with adjustment screw **14** to displace the plunger **13** along the axis of the waveguide **18**. Instead of mechanical adjustment of short-circuiting plane it is possible to use the electrically controlled varactor. The other side of the waveguide **18** has a rod **15** (inhomogeneity) mounted therein between the avalanche diode **8** and the end of the waveguide **18**, inside a channel **16** provided in the wall of the waveguide **18**, to displace the rod **15** in cross-sectional plane of the waveguide **18**. The distance between the axes of avalanche diode **8** and rod **15** is essentially equal to the integer half-wavelength corresponding to the central frequency of radiation generated by the avalanche diode **8**.

Output radiation spectrum position, shape and intensity are determined by a mutual configuration of the avalanche diode, adjustment elements of oscillator chamber , as well as by the current supplied to the avalanche diode.

Operation principle of the device according to the preferred embodiment of invention is as follows. Depending on the selected operation mode, the stabilized current source **6** supplies to the EHF oscillator **1** currents generating either noiselike, or combined radiation. EHF oscillator **1** output is fed through the p-i-n attenuator **2** controlled by the stabilized current source **7** through the impedance transformer **3** to the emitter **4**.

Pulsed amplitude modulation of the EHF radiation is performed by the p-i-n attenuator **2** fed by the voltage source **7** controlled by the microprocessor control unit **5**, providing pulse rate up to 1000 Hz with controlled relative pulse length.

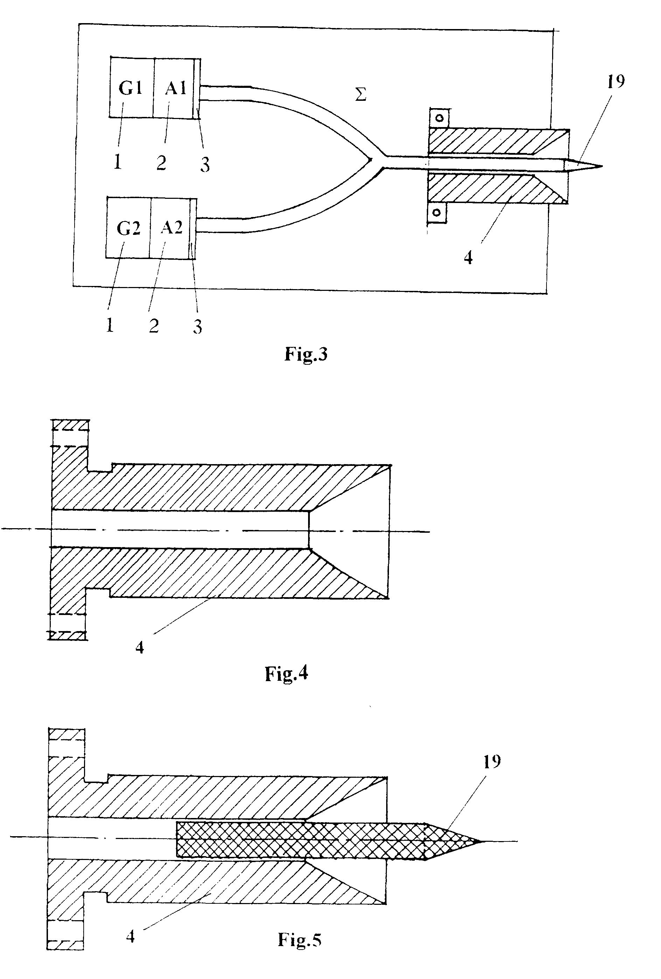

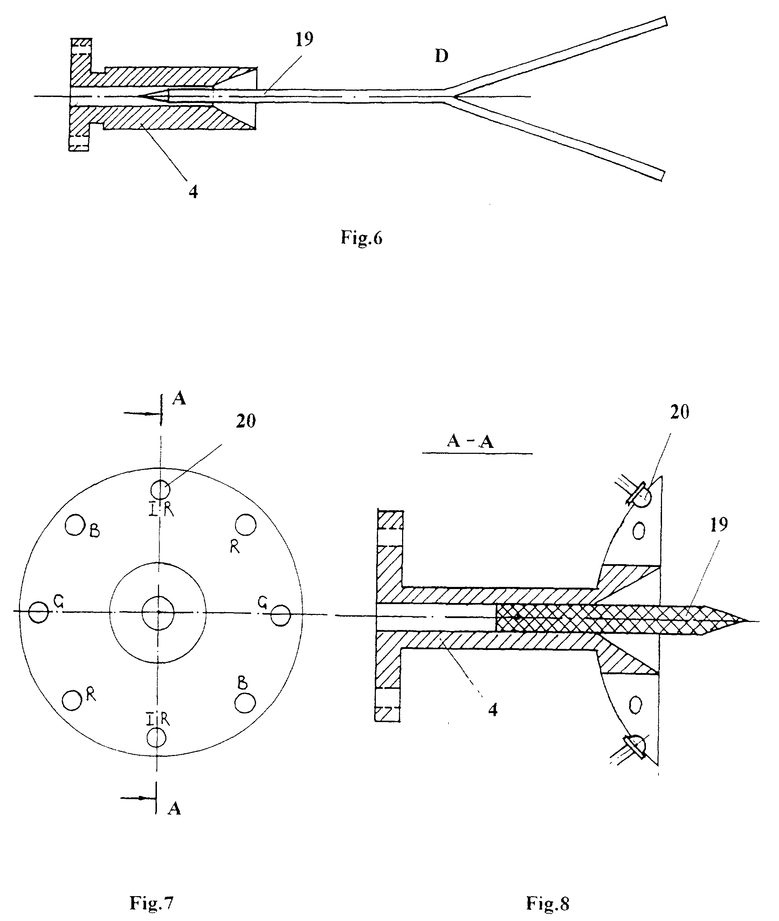

Fig. 4 illustrates the emitter **4** manufactured in form of a horn antenna and additionally provided with:
- dielectric rod antenna **19** mounted therein and having its end surface tapered at an angle equal to the total internal reflection angle, providing thus the focusing of EHF radiation on the selected BAP (Fig. 5);
- flexible dielectric waveguide having EHF power divider **(D)** mounted therein to supply EHF power simultaneously to several BAZes (Fig. 6);
- coherent and/or incoherent sources **20** of infrared **(IR)**, red **(R)**, green **(G)** and blue **(B)** emission (Fig. 7 and Fig. 8).

## Claims

1. A device for EHF puncture including an EHF oscillator whose output is fed to a directed emitter, the said EHF oscillator including a waveguide section with an avalanche diode mounted therein on a heat sink and provided with displacement means in the cross-section plane of the said waveguide, a power-supply post and filter disposed in the wall of the said waveguide, the said waveguide having a movable short-circuiting plunger at one end and inhomogeneity at the other end, wherein a disk resonator (17) is additionally disposed in the said waveguide (18) between the said power-supply post and said avalanche diode (8), **characterised in that** the output of the EHF oscillator is fed through a p-i-n attenuator and impedance transformer, that there is a microprocessor control unit with stabilized current sources mounted therein, and **in that** the inhomogeneity is a single rod, the distance between the axes of the avalanche diode (8) and the rod (15) being essentially equal to the integer half-wavelength corresponding to the central frequency of radiation generated by the avalanche diode (8).

2. A device as provided by the claim 1 **characterised in that** the said device includes two or more EHF radiation oscillators having p-i-n attenuators at their outputs, the outputs of said p-i-n attenuators being connected through the power summator with the said directed emitter.

3. A device as provided by the claim 1 **characterised in that** the said emitter additionally includes a dielectric rod antenna tapered at one end for an angle equal to the total reflection angle.

4. A device as provided by the claim 1 **characterised in that** the said emitter additionally includes a flexible dielectric waweguide with radiation power divider.

5. A device as provided by the claim 1 **characterised in that** the said emitter additionally includes coherent and/or incoherent sources of infrared and visible emission.

## Patentansprüche

1. Vorrichtung zur EHF-Punktion mit einem EHF-Oszillator, dessen Ausgang an einen Richtstrahler geführt ist, wobei der EHF-Oszillator einen Hohlleiterabschnitt aufweist, in dem eine Lawinenlaufzeitdiode auf einem Kühlkörper montiert ist und der mit einer Verstelleinrichtung in der Querschnittsebene des Hohlleiters, einem Stromversorgungsanschluß und einem Filter in der Wand des Hohlleiters versehen ist; der Hohlleiter weist einen verschiebbaren Kurzschlußschieber an einem Ende und eine Inhomogenität am anderen Ende auf, wobei in dem besagten Hohlleiter (18) zwischen dem Stromversorgungsanschluß und der Lawinenlaufzeitdiode (8) zusätzlich ein Scheibenresonator (17) angeordnet ist, **dadurch gekennzeichnet, daß** der Ausgang des EHF-Oszillators über einen pin-Abschwächer und Impedanzwandler geführt ist, eine Mikroprozessorsteuereinheit mit stabilisierten Stromquellen darin montiert ist und die Inhomogenität aus einem einzelnen Stab besteht, wobei der Abstand zwischen den Mittellinien der Lawinenlaufzeitdiode (8) und des Stabes (15) im wesentlichen gleich der ganzzahligen Halbwellenlänge ist, die mit der Mittenfrequenz der von der Lawinenlaufzeitdiode (8) erzeugten Strahlung korrespondiert.

2. Vorrichtung nach Anspruch 1, **dadurch gekennzeichnet, daß** die Vorrichtung zwei oder mehrere Oszillatoren für EHF-Strahlung umfaßt, die an ihren Ausgängen pin-Abschwächer aufweisen, wobei die Ausgänge der pin-Abschwächer über den Leistungsaddierer mit besagtem Richtstrahler verbunden sind.

3. Vorrichtung nach Anspruch 1, **dadurch gekennzeichnet, daß** der besagte Strahler zusätzlich einen dielektrischen Stabstrahler umfaßt, der an einem Ende konisch zulaufend ist, in einem Winkel, der gleich dem Winkel der inneren Totalreflexion ist.

4. Vorrichtung nach Anspruch 1, **dadurch gekennzeichnet, daß** der besagte Strahler zusätzlich einen flexiblen dielektrischen Wellenleiter mit Strahlungsleistungsteiler aufweist.

5. Vorrichtung nach Anspruch 1, **dadurch gekennzeichnet, daß** der besagte Strahler zusätzliche Strahlungsquellen für kohärente und/oder nicht kohärente infrarote und sichtbare Strahlung aufweist.

## Revendications

1. Dispositif pour perforation EHF comprenant un oscillateur EHF dont la sortie alimente un émetteur dirigé, ledit oscillateur EHF comprenant une section de guide d'ondes avec une diode à avalanche montée ici sur un dissipateur thermique et équipée avec une capacité de déphasage dans le plan de coupe dudit guide d'ondes, un bloc d'alimentation électrique et un filtre disposés dans la paroi dudit guide d'ondes, ledit guide d'ondes possédant un plongeur de mise en court-circuit mobile à une extrémité et une inhomogénéité à l'autre extrémité, le résonateur à disque (17) étant placé en outre sur ledit guide d'ondes (18) entre ledit bloc d'alimentation électrique et ladite diode à avalanche (8), **caractérisée en ce que** la sortie de l'oscillateur EHF alimente un atténuateur PIN (Positive Intrinsic Négative) et un transformateur d'impédance, **en ce qu'**il y a une unité de commande à microprocesseur équipée avec des sources de courant stabilisé, et **en ce que** l'inhomogénéité est une tige unique, la distance entre les axes de la diode à avalanche (8) et la tige (15) étant essentiellement égale à la demi-longueur d'onde en nombre entier correspondant à la fréquence centrale du rayonnement produit par la diode à avalanche (8).

2. Dispositif selon la revendication 1 **caractérisé en ce que** ledit dispositif comprend deux ou plusieurs oscillateurs à rayonnement EHF ayant des atténuateurs PIN à leurs sorties, les sorties desdits atténuateurs PIN étant connectées par l'appareil totalisateur de courant avec ledit émetteur dirigé.

3. Dispositif selon la revendication 1 **caractérisé en ce que** ledit émetteur dirigé comprend en outre une antenne diélectrique en forme de tige biseautée à une extrémité selon un angle égal à l'angle total de réflexion.

4. Dispositif selon la revendication 1 **caractérisé en ce que** ledit émetteur comprend en outre un guide d'ondes diélectrique flexible avec un diviseur de puissance de rayonnement.

5. Dispositif selon la revendication 1 **caractérisé en ce que** ledit émetteur comprend en outre des sources cohérentes et/ou incohérentes d'émission infrarouge et visible.
